# EUROPEAN PATENT APPLICATION

(11) **EP 4 725 983 A2**
(43) Date of publication of application: **15.04.2026**
(21) Application number: 26157932.0
(22) Date of filing: 11.06.2020
(51) Int. Cl.: C08J 11/14

(54) **METHOD FOR PRODUCING RECYCLED SUPERABSORBENT POLYMER, METHOD FOR PRODUCING SUPERABSORBENT POLYMER USING RECYCLED SUPERABSORBENT POLYMER, AND RECYCLED SUPERABSORBENT POLYMER**

(30) Priority: 06.09.2019 JP 2019163086
(62) Divisional of application: 20861009.7
(71) Applicant: Unicharm Corporation, Shikokuchuo-shi, Ehime 799-0111 (JP)
(72) Inventor: KONISHI, Takayoshi, Kanonji-shi, 769-1602 (JP); YAMAKI, Kouichi, Kanonji-shi, 769-1602 (JP); KURITA, Noritomo, Kanonji-shi, 769-1602 (JP); TAKAHARA, Takahisa, Minato-ku, 108-8575 (JP)
(74) Representative: Dolleymores

(57) **Abstract**

Provided is a method that is for producing a recycled superabsorbent absorbent polymer and that allows a spent superabsorbent polymer derived from a used sanitary article to be effectively used in a product which uses a superabsorbent polymer as a raw material. This method is for producing from a used superabsorbent polymer obtained from a used sanitary article a recycled superabsorbent polymer that is a raw material for production of a superabsorbent polymer. This method comprises: a deactivation step (S30) for deactivating a used superabsorbent polymer using an acidic solution; an oxidant treatment step (S31) for treating the used superabsorbent polymer deactivated using the acidic solution using an oxidant under acidic conditions; and a drying step (S33) for drying the used superabsorbent polymer treated using the oxidant to generate a recycled superabsorbent polymer.

## Description

### FIELD

The present invention relates to a method of producing a recycled superabsorbent polymer from a used superabsorbent polymer derived from a used sanitary article, a method of producing a superabsorbent polymer using the recycled superabsorbent polymer, and a recycled superabsorbent polymer.

### BACKGROUND

Techniques for recycling used superabsorbent polymers derived from used sanitary articles (for example, absorbent articles) are known. For example, Patent Literature 1 discloses a method of recycling used superabsorbent polymers. The recycling method includes a step of treating used superabsorbent polymers with a polyvalent metal salt aqueous solution and a step of treating the superabsorbent polymers treated with the polyvalent metal salt aqueous solution with an alkali metal salt aqueous solution.

On the other hand, techniques for reusing unused superabsorbent polymers as a part of a raw material of superabsorbent polymers are known. For example, Patent Literature 2 discloses a method of producing resin particles. In the production method, in suspension polymerization, an oily liquid containing a resin b is dispersed in an aqueous dispersion liquid of resin particles A made of a resin a to form resin particles B made of the resin b in the aqueous dispersion liquid and thereby obtain an aqueous dispersion X1 of resin particles C having the resin particles A attached to the surfaces of the resin particles B. At this time, a fine powder removed in a classification step at the time of producing a different lot of the resin particles C is dispersed in the aqueous dispersion liquid. Patent Literature 3 discloses a method of producing a water-absorbing agent. In the production method, a powder containing fine particles of a water-absorbing resin is compressed and shaped in the presence of water, dried and pulverized to produce a water-absorbing agent having an average particle size of 200 to 1000 µm. Patent Literature 4 discloses a method of producing a salt-resistant water-absorbing resin. In the production method, a salt-resistant water-absorbing resin is formed by polymerizing an aqueous solution of at least one monomer component selected from the group consisting of an unsaturated carboxylic acid and a salt thereof in an aqueous solution in a state where a water-absorbing resin is made to be present at a proportion of 1 to 30 parts by weight of the water-absorbing resin per 100 parts by weight of the monomer component. In addition, Non-Patent Literature 1 discloses a method of producing a polyacrylate-based superabsorbent polymer.

### [CITATION LIST]

### [PATENT LITERATURE]

Patent Literature 1: Japanese Unexamined Patent Publication No. 2013-198862
Patent Literature 2: Japanese Unexamined Patent Publication No. 2007-246676
Patent Literature 3: Japanese Unexamined Patent Publication No. H10-204184
Patent Literature 4: Japanese Unexamined Patent Publication No. H4-227705
Patent Literature 5: Japanese Patent No.5996226

### [NON-PATENT LITERATURE]

Non-Patent Literature 1: Tadao Shimomura, "Superabsorbent Polymer", Surface (1991) 495 to 506

### SUMMARY

### [TECHNICAL PROBLEM]

In the recycling of used sanitary articles (for example, absorbent articles), a large number of used sanitary articles are collected, but the types and production companies of a large number of collected used sanitary articles are diverse. Therefore, when superabsorbent polymers derived from a large number of used sanitary articles are collectively recycled by the method of Patent Literature 1, the recycled superabsorbent polymers become mixtures of diverse superabsorbent polymers. That is, the performance of the recycled superabsorbent polymers are less likely to be uniform. Therefore, when recycled superabsorbent polymers are used as they are in sanitary articles for which superabsorbent polymers are used as absorbent materials, there is a risk that it becomes difficult to make the absorption performance of the sanitary articles uniform.

Therefore, the inventors have studied for the first time, for example, the use of recycled superabsorbent polymers as raw materials (fine powder, fine particles, or water-absorbing resin) of Patent Literature 2 to 4, or as raw materials or semi-product of Non-Patent Literature 1, in the methods of producing superabsorbent polymers in Patent Literature 2 to 4 or Non-Patent Literature 1. In the production methods of Patent Literature 2 to 4 or Non-Patent Literature 1, it is considered that unused raw materials or the like (for example, superabsorbent polymers) containing an extremely small amount of an impurity is used. Incidentally, in recycled superabsorbent polymers derived from used sanitary articles, there are cases where unique impurities (for example, an odorous substance, a colored substance, and bacteria) are present. Therefore, when recycled superabsorbent polymers are used in the production methods of Patent Literature 2 to 4 or Non-Patent Literature 1 in which unused superabsorbent polymers containing an extremely small amount of an impurity are used, there is a risk that highly pure high-quality superabsorbent polymers cannot be formed. In such a case, there is a risk that superabsorbent polymers produced by the production methods of Patent Literature 2 to 4 or Non-Patent Literature 1 using recycled superabsorbent polymers cannot be used for sanitary articles for which superabsorbent polymers are used.

An aspect of the present invention is to provide a method of producing a recycled superabsorbent polymer which makes it possible to effectively use a used superabsorbent polymer derived from a used sanitary article for products for which a superabsorbent polymer is used as a raw material, a method of producing a superabsorbent polymer using a recycled superabsorbent polymer, and a recycled superabsorbent polymer.

### [SOLUTION TO PROBLEM]

The present invention is a method of producing recycled superabsorbent polymer that is a raw material for producing a superabsorbent polymer from a used superabsorbent polymer derived from a used sanitary article, the method comprising: an inactivation step of inactivating the used superabsorbent polymer with an acidic solution; an oxidizing agent treatment step of treating the used superabsorbent polymer inactivated with the acidic solution with an oxidizing agent under an acidic condition; and a drying step of drying the used superabsorbent polymer treated with the oxidizing agent to generate the recycled superabsorbent polymer.

The present invention is a method of producing a superabsorbent polymer using a recycled superabsorbent polymer that is recycled from a used superabsorbent polymer derived from a used sanitary article and is a raw material for producing a superabsorbent polymer, the method comprising: a step of producing the recycled superabsorbent polymer by the above-described method; and a step of executing aqueous solution polymerization or crosslinking for producing the superabsorbent polymer by incorporating the recycled superabsorbent polymer into at least one of a raw material and a semi-product for the production in the aqueous solution polymerization or crosslinking.

The present invention is a recycled superabsorbent polymer that is recycled from a used superabsorbent polymer derived from a used sanitary article and is a raw material for producing a superabsorbent polymer, in which an ash content is 35 mass% or less and a viable bacteria count detected by a pour culture method is a detection limit or less.

### [ADVANTAGEOUS EFFECT OF THE INVENTION]

According to the present invention, it becomes possible to provide a method of producing a recycled superabsorbent polymer which makes it possible to effectively use a used superabsorbent polymer derived from a used sanitary article for products for which a superabsorbent polymer is used as a raw material, a method of producing a superabsorbent polymer using a recycled superabsorbent polymer, and a recycled superabsorbent polymer.

### BRIEF DESCRIPTION OF DRAWINGS

FIG. 1 is a block diagram illustrating a configuration example of a system that is used in a method of producing recycled superabsorbent polymers according to an embodiment.
FIG. 2 is a flow chart illustrating an example of the method of producing recycled superabsorbent polymers according to the embodiment.
FIG. 3 is a block diagram illustrating a configuration example of an apparatus for extracting used superabsorbent polymers according to the embodiment.
FIG. 4 is a flow chart illustrating an example of a method of extracting used superabsorbent polymers according to the embodiment.

### DESCRIPTION OF EMBODIMENTS

The present embodiment relates to the following aspects.

### [Aspect 1]

A method of producing a recycled superabsorbent polymer that is a raw material for producing a superabsorbent polymer from a used superabsorbent polymer derived from a used sanitary article, the method comprising:
an inactivation step of inactivating the used superabsorbent polymer with an acidic solution;
an oxidizing agent treatment step of treating the used superabsorbent polymer inactivated with the acidic solution with an oxidizing agent under an acidic condition; and
a drying step of drying the used superabsorbent polymer treated with the oxidizing agent to generate the recycled superabsorbent polymer.

In the present method, a used superabsorbent polymer derived from a used sanitary article (for example, an absorbent article) is inactivated with an acidic solution (inactivation step) and then treated with an oxidizing agent under an acidic condition (oxidizing agent treatment step). That is, in the present method, a used superabsorbent polymer is dehydrated with an acidic solution to significantly decrease the volume of the used superabsorbent polymer, and then the used superabsorbent polymer is deodorized, decolorized, and sterilized with an oxidizing agent under an acidic condition while maintaining the dehydrated state, that is, the state where the volume is decreased. Such a decrease in the volume of the used superabsorbent polymer makes it possible to easily remove impurities (for example, an odorous substance, a colored substance, and bacteria) that are contained in the recycled superabsorbent polymer derived from a used sanitary article with an oxidizing agent and makes it possible to obtain a highly pure recycled superabsorbent polymer. Such a highly pure recycled superabsorbent polymer can be used as a part of a raw material in a predetermined method for forming a superabsorbent polymer to form highly pure high-quality superabsorbent polymers. Examples of the predetermined method of producing a superabsorbent polymer include methods as in Patent Literature 2 to 4 and a method in which a superabsorbent polymer is produced using a recycled superabsorbent polymer described below, that is, a method in which an unused superabsorbent polymer is reused as a part of a raw material or a semi-product of the superabsorbent polymer. This makes it possible to effectively use the used superabsorbent polymer derived from a used sanitary article for products for which a superabsorbent polymer is used.

### [Aspect 2]

The method according to aspect 1, further comprising:
a foreign matter separation step of separating a foreign matter from the recycled superabsorbent polymer after the drying step.

In the present method, a foreign matter (for example, a pulp fiber) is separated from the dried recycled superabsorbent polymer. Therefore, compared with separation in a non-dry state such as separation in a solution, the foreign matter can be relatively easily separated from the recycled superabsorbent polymer, which makes it possible to obtain a more highly pure recycled superabsorbent polymer.

### [Aspect 3]

The method according to aspect 1 or 2, wherein
the acidic solution is a solution containing citric acid,
the method further comprising, after the inactivation step and before the drying step: a citric acid removal step of removing the citric acid in the used superabsorbent polymer.

In the present method, the used superabsorbent polymer is inactivated with a solution containing citric acid (inactivation step). This does not only simply dehydrate the used superabsorbent polymer to significantly decrease the volume of the used superabsorbent polymer, but also makes it possible to easily remove a metal (for example, a metal derived from the human body that is contained in excrement) by a chelating effect of citric acid. Furthermore, in the present method, the citric acid in the used superabsorbent polymer is removed after the inactivation step (citric acid removal step). This makes it possible to significantly suppress a chelating agent such as citric acid remaining in the recycled superabsorbent polymer. These make it possible to obtain a more highly pure recycled superabsorbent polymer. In particular, the chelating agent rarely remains in the recycled superabsorbent polymer, which makes it possible to significantly suppress the chelating agent significantly impairing a crosslinking reaction in a case where aqueous solution polymerization, crosslinking, or the like is performed in a method in which an unused superabsorbent polymer is reused as a part of a raw material of a superabsorbent polymer (for example, Patent Literature 2 and 4).

### [Aspect 4]

The method according to aspect 3, wherein
the citric acid removal step includes a removal treatment step of treating the used superabsorbent polymer with an acid for removal that is an acid that does not form a chelating structure with a metal ion, an organic solvent that is miscible with water, or an aqueous solution thereof.

In the present method, as the removal of the citric acid, the used superabsorbent polymer is treated with an acid for removal that does not form a chelating structure with a metal ion, an organic solvent that is miscible with water, or an aqueous solution thereof. This makes it possible to wash off the citric acid on a surface of the used superabsorbent polymer while dehydrating the used superabsorbent polymer with the acid or the organic solvent. This makes it possible to more significantly suppress the chelating agent such as citric acid remaining in the recycled superabsorbent polymer.

### [Aspect 5]

The method according to aspect 4, wherein
the removal treatment step includes, during or after the oxidizing agent treatment step, an acid step of treating the used superabsorbent polymer with the acid for removal or an aqueous solution thereof.

In the present method, during or after the oxidizing agent treatment step, the used superabsorbent polymer is treated with the acid for removal or an aqueous solution thereof. This makes it possible to further dehydrate the used superabsorbent polymer while removing the citric acid from the used superabsorbent polymer. This makes it possible to more easily execute the subsequent drying step and thereby makes it possible to efficiently obtain a highly pure recycled superabsorbent polymer.

### [Aspect 6]

The method according to aspect 4 or 5, wherein
the acid for removal is an acid having no carboxyl group or an acid having one carboxyl group in one molecule.

In the present method, the acid for removal is an acid having no carboxyl group or an acid having one carboxyl group in one molecule. This makes it possible in the removal treatment step to further dehydrate the used superabsorbent polymer while removing the citric acid from the used superabsorbent polymer.

### [Aspect 7]

The method according to any one of aspects 4 to 6, wherein
the removal treatment step includes, after the oxidizing agent treatment step and before the drying step, an organic solvent step of treating the used superabsorbent polymer with an aqueous solution of the organic solvent.

In the present method, the used superabsorbent polymer is treated with an aqueous solution of the organic solvent after the oxidizing agent treatment step and before the drying step. This makes it possible to further dehydrate the used superabsorbent polymer while removing the citric acid from the used superabsorbent polymer. This makes it possible to more easily execute the subsequent drying step and thereby makes it possible to efficiently obtain a highly pure recycled superabsorbent polymer.

### [Aspect 8]

The method according to aspect 7, wherein
the organic solvent step includes a step of treating the used superabsorbent polymer with an aqueous solution of the organic solvent containing an alkali metal ion source capable of supplying an alkali metal ion.

In the present method, the used superabsorbent polymer is treated with an aqueous solution of an organic solvent containing an alkali metal ion source. This makes it possible to neutralize the used superabsorbent polymer with the alkali metal ion while removing the citric acid from the used superabsorbent polymer and dehydrating the used superabsorbent polymer. This makes it possible to use the recycled superabsorbent polymer as a neutralized highly pure raw material at the time of, for example, producing a superabsorbent polymer.

### [Aspect 9]

The method according to aspect 8, wherein
the alkali metal ion source is a hydroxide of an alkali metal, or a salt of a hydroxide of an alkali metal and an acid having a larger acid dissociation constant than an acid group of the superabsorbent polymer.

In the present method, the alkali metal ion source is a hydroxide of an alkali metal, or a salt of a hydroxide of an alkali metal and an acid having a larger acid dissociation constant than an acid group of the superabsorbent polymer. This makes it possible to more reliably neutralize the used superabsorbent polymer with the alkali metal ion.

### [Aspect 10]

The method according to any one of aspects 1 to 9, wherein
in the oxidizing agent treatment step, the oxidizing agent contains water containing ozone or a hydrogen peroxide solution.

In the present method, the used superabsorbent polymer is treated with water containing ozone or a hydrogen peroxide solution. This makes it possible to more easily remove impurities (for example, an odorous substance, a colored substance, and bacteria) contained in the recycled superabsorbent polymer derived from a used sanitary article, that is, makes it possible to more easily perform deodorization, decoloration, and sterilization. This makes it possible to obtain more highly pure recycled superabsorbent polymer.

### [Aspect 11]

A method of producing a superabsorbent polymer using a recycled superabsorbent polymer that is recycled from a used superabsorbent polymer derived from a used sanitary article and is a raw material for producing a superabsorbent polymer, the method comprising:
a step of producing the recycled superabsorbent polymer by the method according to any one of aspects 1 to 10; and
a step of executing aqueous solution polymerization or crosslinking for producing the superabsorbent polymer by incorporating the recycled superabsorbent polymer into at least one of a raw material and a semi-product for the production in the aqueous solution polymerization or crosslinking.

In the present method, a recycled superabsorbent polymer derived from a used sanitary article (for example, an absorbent article) is incorporated into at least one of a raw material and a semi-product, and a superabsorbent polymer is produced by aqueous solution polymerization or crosslinking. At this time, since the recycled superabsorbent polymer is produced by the method of aspects 1 to 10, the purity thereof becomes high. This makes it possible in the present method to form highly pure high-quality superabsorbent polymer even when a recycled superabsorbent polymer is used.

### [Aspect 12]

A recycled superabsorbent polymer that is recycled from a used superabsorbent polymer derived from a used sanitary article and is a raw material for producing a superabsorbent polymer, in which an ash content is 35 mass% or less and a viable bacteria count detected by a pour culture method is a detection limit or less.

In the present recycled superabsorbent polymers derived from a used sanitary article (for example, an absorbent article), the ash content is 35 mass% or less, and the viable bacteria count detected by a pour culture method is a detection limit or less. Here, it is considered that the amount of ash contained inside an ordinary superabsorbent polymer that is not used after production is about 30 mass% and that the amount of ash contained inside the recycled superabsorbent polymer is also about 30 mass%. Therefore, there is only a small amount of unnecessary ash (5 mass% or less) on the surfaces of the recycled superabsorbent polymer, and the recycled superabsorbent polymer has an extremely small viable bacteria count (the detection limit or less). Therefore, the purity of the recycled superabsorbent polymer becomes extremely high. This makes it possible to form a highly pure high-quality superabsorbent polymer by incorporating such a recycled superabsorbent polymer into at least one of a raw material and a semi-product in suspension polymerization, aqueous solution polymerization, or crosslinking for producing the superabsorbent polymer.

Hereinafter, a method of producing a recycled superabsorbent polymer that is a raw material for producing a superabsorbent polymer from a used superabsorbent polymer derived from a used sanitary article, a method for producing a superabsorbent polymer using the recycled superabsorbent polymer, and a recycled superabsorbent polymer according to an embodiment will be described below. In the present specification, the sanitary article refers to an article that contributes to sanitation and contains superabsorbent polymers. The used sanitary article (for example, an absorbent article) refers to a sanitary article used by a user and mainly refers to a sanitary article that has absorbed and held a liquid substance released from the user (for example, excrement), and includes a sanitary article that has been used but has not absorbed and held a liquid substance or a sanitary article that is not used but has been disposed of. The used superabsorbent polymers refer to superabsorbent polymers contained in a used sanitary article. The recycled superabsorbent polymers refer to superabsorbent polymers recycled from used superabsorbent polymers derived from a used sanitary article. In the present embodiment, an absorbent article will be described as an example of the sanitary article. Examples of the absorbent article include a paper diaper, an incontinence pad, a sanitary napkin, a bed sheet, and a pet sheet, in which superabsorbent polymers are included, and pulp fibers may be further included.

First, a configuration example of the absorbent article will be described. The absorbent article includes a top sheet, a back sheet, and an absorbent body arranged between the top sheet and the back sheet. The size of the absorbent article is, for example, about 15 to 100 cm in length and 5 to 100 cm in width. It should be noted that the absorbent article may further include a different member that ordinary absorbent articles include, for example, a diffusion sheet, leakproof walls, side sheets, or the like.

Examples of a configuration member of the top sheet include a liquid-permeable nonwoven fabric, a synthetic resin film having a liquid-permeating hole, a composite sheet thereof, and the like. Examples of a configuration member of the back sheet include a liquid-impermeable nonwoven fabric, a liquid-impermeable synthetic resin film, and a composite sheet thereof. Examples of a configuration member of the diffusion sheet include a liquid-permeable nonwoven fabric. Examples of a configuration member of the leakproof wall or the side sheet include a liquid-impermeable nonwoven fabric, and the leakproof wall may include an elastic member such as rubber. The material of the nonwoven fabric or the synthetic resin film is not particularly limited as long as the material can be used as an absorbent article, and examples thereof include an olefin-based resin such as polyethylene or polypropylene, a polyamide-based resin such as 6-nylon or 6,6-nylon, a polyester-based resin such as polyethylene terephthalate (PET) or polybutylene terephthalate (PBT), and the like. As the material of the nonwoven fabric, natural fibers such as cotton or rayon may be used. In the present embodiment, an absorbent article for which a film is used as the configuration member of the back sheet and a nonwoven fabric is used as the configuration member of the top sheet will be described as an example.

Examples of the configuration member of the absorbent body include absorbent body materials, that is, a pulp fiber and a superabsorbent polymer. The pulp fiber is not particularly limited as long as the pulp fiber can be used as an absorbent article, and examples thereof include cellulose-based fibers. Examples of the cellulose-based fibers include a wood pulp, a crosslinked pulp, a non-wood pulp, a recycled cellulose, a semi-synthetic cellulose, and the like. As the size of the pulp fiber, the average value of the long diameters of the fibers is, for example, several tens of micrometer and preferably 20 to 40 µm, and the average value of the fiber lengths is, for example, several millimeters and preferably 2 to 5 mm. The superabsorbent polymer (SAP) is not particularly limited as long as the superabsorbent polymer can be used as an absorbent article, and examples thereof include polyacrylate-based, polysulfonate-based, and maleic anhydride-based water-absorbing polymers. As the size of the (dried) superabsorbent polymer, the average value of the particle sizes is, for example, several hundred micrometers and preferably 200 to 500 µm. The absorbent body may include a core wrap formed of a liquid-permeable sheet.

One surface and the other surface of the absorbent body are joined to the top sheet and the back sheet, respectively, via an adhesive. In a plan view, a portion (peripheral portion) of the top sheet which extends toward the outer side of the absorbent body so as to surround the absorbent body is joined to a portion (peripheral portion) of the back sheet which extends toward the outer side of the absorbent body so as to surround the absorbent body, via an adhesive. Therefore, the absorbent body is wrapped inside the joined body of the top sheet and the back sheet. The adhesive is not particularly limited as long as the adhesive can be used as an absorbent article, and examples thereof include a hot-melt adhesive. Examples of the hot-melt adhesive include pressure-sensitive adhesives or heat-sensitive adhesives mainly containing rubber such as styrene-ethylene-butadiene-styrene, styrene-butadiene-styrene, or styrene-isoprene-styrene or mainly containing an olefin such as polyethylene.

Next, the method of producing recycled superabsorbent polymers that are raw materials for producing superabsorbent polymers from used superabsorbent polymers derived from a used sanitary article according to the embodiment will be described.

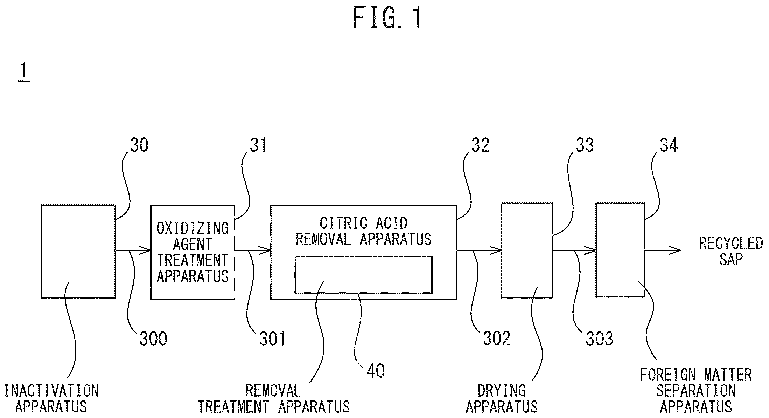

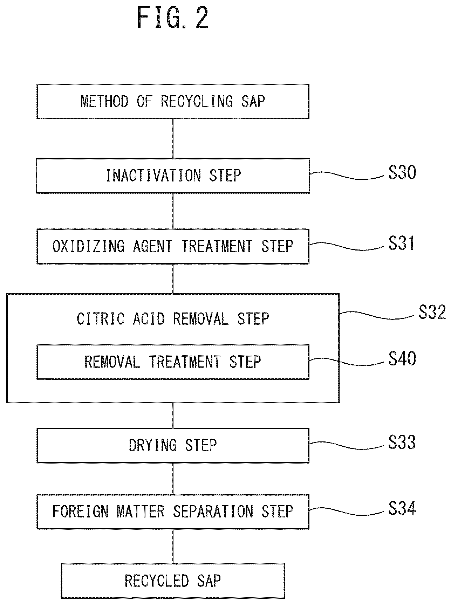

FIG. 1 is a block diagram illustrating a configuration example of a system 1 that is used in the method of producing recycled superabsorbent polymers according to the embodiment. FIG. 2 is a flow chart illustrating an example of the method of producing recycled superabsorbent polymers according to the embodiment.

The method of producing recycled superabsorbent polymers includes an inactivation step S30, an oxidizing agent treatment step S31, and a drying step S33 and may further include a foreign matter separation step S34. In a case where citric acid is used in the inactivation step S30 or the like, a citric acid removal step S32 may further be provided. In such a production method, the citric acid removal step S32 may include a removal treatment step S40. Incidentally, the system 1 used in the method of producing recycled superabsorbent polymers includes an inactivation apparatus 30, an oxidizing agent treatment apparatus 31, and a drying apparatus 33 and may further include a foreign matter separation apparatus 34. In a case where citric acid is used in the inactivation apparatus 30 or the like, a citric acid removal apparatus 32 may further be provided. In such a system 1, it is preferable that the citric acid removal apparatus 32 may include a removal treatment apparatus 40. Hereinafter, each step will be specifically described.

The inactivation step S30 is executed with the inactivation apparatus 30. In the inactivation step S30, used superabsorbent polymers are inactivated with an acidic solution. In the present embodiment, used superabsorbent polymers extracted from a used absorbent article are immersed in an acidic aqueous solution that is an inactivating agent, thereby forming inactivated superabsorbent polymers 300.

An acid in the acidic aqueous solution is not particularly limited, and examples thereof include an inorganic acid and an organic acid. When the superabsorbent polymers are inactivated with an acid, it is difficult to leave ash in the superabsorbent polymers and the pulp fibers compared with a case where the superabsorbent polymers are inactivated with lime, calcium chloride, or the like. Examples of the inorganic acid include sulfuric acid, hydrochloric acid, and nitric acid, and sulfuric acid is preferable from the viewpoint of not containing chlorine or the viewpoint of costs or the like. Examples of the organic acid include a carboxylic acid having a plurality of carboxyl groups in one molecule (for example, citric acid, tartaric acid, malic acid, succinic acid, or oxalic acid), a carboxylic acid having one carboxyl group in one molecule (for example, gluconic acid, pentanoic acid, butanoic acid, propionic acid, glycolic acid, acetic acid, or formic acid), a sulfonic acid (for example, methanesulfonic acid, trifluoromethanesulfonic acid, benzenesulfonic acid, or p-toluenesulfonic acid), and the like. As the organic acid, an organic acid having a plurality of carboxyl groups is preferable and citric acid is more preferable from the viewpoint of likelihood of forming a chelate complex with a divalent or higher metal (for example, calcium) contained in excrement or the like and a difficulty in leaving ash in the superabsorbent polymers and the pulp fibers. The citric acid concentration of the acidic aqueous solution is not particularly limited and is, for example, 0.5 to 4 mass%. In the present embodiment, citric acid is used as the acid in the acidic aqueous solution.

The acidic aqueous solution needs to be acidic and preferably has a predetermined pH. The upper limit of the predetermined pH is preferably 4.0, more preferably 3.5, and still more preferably 3.0. When the predetermined pH is too high, since the superabsorbent polymers are not sufficiently inactivated, there is a tendency that discharge of excrement held by the superabsorbent polymers becomes insufficient, and there is a tendency that separation from the pulp fibers or the like becomes difficult. On the other hand, the lower limit of the predetermined pH is preferably 0.5 and more preferably 1.0. When the predetermined pH is too low, since the superabsorbent polymers are further inactivated, and, in the removal treatment step S40 (described below), there is a tendency that a reaction between the inactivated superabsorbent polymers and an aqueous solution containing the alkali metal ion source takes a long time. In addition, when the predetermined pH is too low, in the case of recycling recycled pulp fibers in addition to recycling superabsorbent polymers derived from a used absorbent article, there is a risk that the recycled pulp fibers are damaged. It should be noted that, in the present specification, the pH means a value at 25°C. In addition, the pH can be measured using, for example, a twin pH meter AS-711 manufactured by HORIBA Co., Ltd. In the method of producing recycled superabsorbent polymers, it is preferable that the above-described predetermined pH is satisfied at the end of the inactivation step S30. This is because the superabsorbent polymers are continuously inactivated.

The specific configuration of the inactivation apparatus 30 that executes the inactivation step S30 is not particularly limited as long as the superabsorbent polymers can be immersed in the acidic aqueous solution. The inactivation apparatus 30 has, for example, a tank in which members including the superabsorbent polymers can be arranged and the acidic aqueous solution can be stored. In the inactivation step S30, the members including the superabsorbent polymers may be put into the tank after the acidic aqueous solution is stored in the tank or the acidic aqueous solution may be put into the tank after the members including the superabsorbent polymers are arranged in the tank. The members including the superabsorbent polymers are immersed in the acidic aqueous solution and an inactivation reaction is caused.

In the inactivation step S30, in order to evenly progress the reaction, while also depending on temperatures, the superabsorbent polymers can be inactivated by stirring the members including the superabsorbent polymers (for example, the used absorbent article) in the tank containing the acidic aqueous solution for, for example, about 5 to 60 minutes. In the inactivation step S30, the temperature of the acidic aqueous solution is not particularly limited and is, for example, room temperature (25°C), preferably a temperature higher than room temperature, more preferably 60°C to 95°C, and still more preferably 70°C to 90°C. This makes it easy to sterilize bacteria derived from excrement or the like that is contained in the acidic aqueous solution with the acid in the acidic aqueous solution.

In the end stage of the treatment of the inactivation step S30, the superabsorbent polymers 300 are not completely dehydrated, are in a state of having absorbed moisture, while not much (for example, a state of having absorbed water as much as about 10 times the volume of the superabsorbent polymers before the absorption of water), and is in a gel state.

It should be noted that, in a case where the members to be immersed in the acidic aqueous solution includes, in addition to the superabsorbent polymers, different members such as pulp fibers, a liquid-permeable sheet, and a liquid-impermeable sheet, a separation step of separating and removing the members other than the superabsorbent polymers may be executed before or after the inactivation step S30. The separation step may be executed in parallel with the inactivation step S30 at the same time. Examples of the case where the members to be immersed in the acidic aqueous solution includes, in addition to the superabsorbent polymers, different members include a case where the member is a used absorbent article containing superabsorbent polymers. The separation step will be described below.

After that, the inactivated superabsorbent polymers are separated from the acidic aqueous solution (solid-liquid separation) with a sieve (or mesh) provided in the inactivation apparatus 30 and then supplied to the oxidizing agent treatment step S31 (oxidizing agent treatment apparatus 31) as the superabsorbent polymers 300.

The oxidizing agent treatment step S31 is executed with the oxidizing agent treatment apparatus 31. In the oxidizing agent treatment step S31, the superabsorbent polymers 300 inactivated with the acidic solution are treated with an oxidizing agent under an acidic condition. The oxidizing agent is not particularly limited as long as the oxidizing agent is capable of removing impurities (for example, an odorous substance, a colored substance, and bacteria) that are attached to the surfaces of the superabsorbent polymers 300, and examples thereof include ozone and hydrogen peroxide. In a case where the oxidizing agent is incorporated into water and used as an aqueous solution, the concentration of the oxidizing agent in the aqueous solution is, for example, 0.5 to 20 mass%. In the present embodiment, superabsorbent polymers 301 from which impurities on the surfaces of the superabsorbent polymers 300 have been removed are formed by bringing the inactivated superabsorbent polymers 300 into contact with (immersing) an aqueous solution of the oxidizing agent, that is, ozone water containing a predetermined concentration of ozone, for a predetermined time. In addition, the acidic condition needs to be acidic, and examples thereof include an aqueous solution exhibiting acidity. As the aqueous solution exhibiting acidity, an aqueous solution containing an inorganic acid or an organic acid exemplified in the above description of the acidic aqueous solution is preferable from the viewpoint of maintaining the inactivated state of the superabsorbent polymers 300, the viewpoint of making it difficult to deactivate ozone in the case of using ozone, or the like. In addition, the pH of the aqueous solution exhibiting acidity is in a predetermined range, that is, at least the same as the pH of the above-described acidic aqueous solution (preferably 0.5 to 4.0 and more preferably 1.0 to 3.5). In the present embodiment, an aqueous solution of citric acid is used as the acidic condition. The citric acid concentration of the aqueous solution is not particularly limited and is, for example, 0.1 to 5 mass%.

To the surfaces of the superabsorbent polymers 300, bacteria, other organic substances, or the like derived from excrement in the used absorbent article are attached as a foreign matter or dirt. When bacteria, other organic substances, or the like remain on the surfaces, there is a risk that the purity of the recycled superabsorbent polymers decreases. Therefore, in the oxidizing agent treatment step S31, the superabsorbent polymers 300 are substantially uniformly dispersed in an aqueous solution of the oxidizing agent, in the present embodiment, in ozone water, at a predetermined concentration (for example, 1 to 10 mass%), and bacteria, other organic substances, or the like attached to the surfaces of the superabsorbent polymers 300 are oxidatively decomposed and removed. That is, in the oxidizing agent treatment step S31, impurities that are attached to the surfaces of the superabsorbent polymers 300 are removed. In particular, sterilization with ozone water is referred to as bacteriolysis, and a chemical reaction between protein and ozone breaks the cell walls (membranes) of bacteria, and components in the cells leak, whereby the bacteria are killed. Therefore, the sterilization effect is significantly strong, resistant bacteria are less likely to be generated, and the possibility of bacteria being propagated again after sterilization and covering the surfaces is extremely low.

At this time, when the superabsorbent polymers are inactivated with the acidic aqueous solution (inactivation step S30), the inactivated superabsorbent polymers do not absorb water. Therefore, the superabsorbent polymers can be washed with ozone water having a low ozone concentration in an inactivated state, which makes it easy to remove bacteria and the like by washing and makes it possible to remove the bacteria and the like within a short period of time. Furthermore, since an odor component and a pigment component are also decomposed by ozone, there is also a deodorization and decolorization effect. It should be noted that, when the superabsorbent polymers 300 are treated with a gaseous substance, for example, ozone gas, since the superabsorbent polymers 300 are in a massive form (gel form), the inside of the mass is not properly treated, and it becomes difficult to uniformly treat the superabsorbent polymers as a whole. When the superabsorbent polymers are treated with ultraviolet rays or radioactive rays at a dose strong enough for the ultraviolet rays or radioactive rays to permeate the inside of the mass, the superabsorbent polymers are decomposed. When the superabsorbent polymers are treated by heating or with high-pressure steam, dirt of excrement remains in the superabsorbent polymers. When the superabsorbent polymers are treated with an aqueous solution of sodium hypochlorite, since chlorine remains in the superabsorbent polymers, the superabsorbent polymers cannot be used as a sanitary material.

The ozone concentration in the ozone water is not particularly limited as long as bacteria, other organic substances, or the like attached to the surfaces of the superabsorbent polymers can be removed, but is preferably 0.3 to 2 ppm by mass and more preferably 0.5 to 1.5 ppm by mass. When the concentration is too low, the removal of bacteria or the like becomes difficult, and, when the concentration is too high, there is a risk that the superabsorbent polymers may begin to decompose. The contact time between the ozone water and the superabsorbent polymers is not particularly limited as long as bacteria, other organic substances, or the like attached to the surfaces of the superabsorbent polymers can be removed. The contact time is set to be short when the ozone concentration in the ozone water is high and set to be long when the ozone concentration is low. The contact time is preferably 0.3 seconds to 15 minutes and more preferably 5 seconds to 10 minutes. The product of the ozone concentration (ppm) in the ozone water and the contact time (min) (hereinafter, also referred to as "CT value") is preferably 0.05 to 20 ppm·min and more preferably 0.08 to 10 ppm·min. When the CT value is too small, sterilization becomes difficult, and, when the CT value is too large, there is a risk that the superabsorbent polymers may decompose. Here, in the treatment with the ozone water, not only the removal of bacteria or the like but also bleaching can be performed by removing the impurities attached to the surfaces of the superabsorbent polymers 300. Examples of an ozone generation apparatus that supplies ozone to water include an ozone water exposure tester ED-OWX-2 manufactured by Ecodesign Co., Ltd., an ozone generation apparatus OS-25V manufactured by Mitsubishi Electric Corporation, and the like.

In the oxidizing agent treatment step S31, in order to evenly progress the reaction, the ozone water containing the superabsorbent polymers 300 may be stirred in the tank. In the oxidizing agent treatment step S31, the temperature of the ozone water is not particularly limited and is, for example, room temperature (25°C) and preferably 10°C to 40°C. When the temperature of the ozone water is set to be too high, ozone is likely to escape as gas and is likely to be deactivated, and, when the temperature is set to be too low, the time of the ozone treatment is likely to become long. This makes it easy to remove bacteria or organic substances attached to the surfaces of the superabsorbent polymers 300 with ozone in the ozone water.

The specific configuration of the oxidizing agent treatment apparatus 31 that executes the oxidizing agent treatment step S31 is not particularly limited as long as the inactivated superabsorbent polymers 300 can be brought into contact with (immersed in) the ozone water without breaking the shape. Examples of the oxidizing agent treatment apparatus 31 include a twin screw pump (BQ-type: manufactured by Fukko Kinzoku Industry Co., Ltd.). The twin screw pump is a positive displacement self-priming pump. The twin screw pump includes a casing and twin screws that are arranged in a room in the casing and extend parallel to each other. The ozone water containing the superabsorbent polymers 300 is supplied to the room, reaches the twin screws in the radial direction, then, is extruded in the axial direction by the rotation of the twin screws, and discharged. In the stage of the treatment of the oxidizing agent treatment step S31, since the superabsorbent polymers 300 are in a state of a gel having absorbed moisture, while not much, as described above, it is difficult to stir the gel with a stirring blade in a manner that the gel does not break and to evenly progress the reaction. The twin screw pump is preferable since the twin screw pump is capable of evenly mixing the inactivated superabsorbent polymers 300 with the ozone water without any stirring blades while rarely shearing the superabsorbent polymers 300 and thus not breaking the gel. In the oxidizing agent treatment step S31, the inactivated superabsorbent polymers 300 and the ozone water are mixed and reacted with each other with the twin screw pump.

After that, the ozone-treated superabsorbent polymers 300 are separated from the ozone water (solid-liquid separation) with a sieve (or mesh) provided in the oxidizing agent treatment apparatus 31 and then supplied to the citric acid removal step S32 (citric acid removal apparatus 32) as the superabsorbent polymers 301.

The citric acid removal step S32 is executed with the citric acid removal apparatus 32. In a case where the acidic solution used in the inactivation step S30 is a solution containing citric acid, in the citric acid removal step S32, the citric acid in the used superabsorbent polymers 301 is removed after the inactivation step S30 and before the drying step S33. In the present embodiment, in the inactivation step S30, a solution containing citric acid is used as the acidic solution, which makes it possible not only to inactivate the used superabsorbent polymers, but also to easily remove a metal attached to the surfaces of the used superabsorbent polymers due to the chelating effect of citric acid. In addition, in the citric acid removal step S32, a chelating agent such as citric acid remaining in the finally obtained recycled superabsorbent polymers can be significantly suppressed.

Here, the citric acid removal step S32 includes a removal treatment step S40 (removal treatment apparatus 40) of treating the used superabsorbent polymers 301 with an acid for removal that is an acid that does not form a chelating structure with a metal ion, an organic solvent that is miscible with water, or an aqueous solution thereof. This makes it possible to further dehydrate the used superabsorbent polymers while melting the citric acid into a predetermined acid or organic solvent from the used superabsorbent polymers to remove the citric acid.

As the acid for removal that is an acid that does not form a chelating structure with a metal ion, for example, among the inorganic acids and the organic acids exemplified in the above description of the acidic aqueous solution, an acid having no carboxyl group or an acid having one (not a plurality of) carboxyl group in one molecule is exemplified. In other words, the acid for removal can be referred to as, among the inorganic acids and the organic acids, an acid not having a plurality of carboxyl groups in one molecule. Therefore, in a case where the acid for removal remains on the surfaces of the used superabsorbent polymers, the acid for removal does not function as a chelating agent.

In this case, the removal treatment step S40 can be said to include an acid step of treating the used superabsorbent polymers with an acid for removal or an aqueous solution thereof after the oxidizing agent treatment step S31 and before the drying step S33.

Incidentally, the organic solvent that is miscible with water is not particularly limited, and examples thereof include alcohol-based solvents (for example, methanol, ethanol, propyl alcohol, an isomer thereof, butyl alcohol, and an isomer thereof), ketone-based solvents (for example, acetone or methyl ethyl ketone), nitrile-based solvents (for example, acetonitrile), and the like. This makes it possible to further dehydrate and wash the used superabsorbent polymers. In the present embodiment, in the removal treatment step S40 of the citric acid removal step S32, methanol, which is an organic solvent that is miscible with water, is used.

The organic solvent may contain a different solvent, for example, water as long as the removal of the citric acid is not affected. The proportion of the different solvent is less than 50 mass%, preferably 10 mass% or less and more preferably 0 mass% with respect to the organic solvent.

In this case, the removal treatment step S40 can be said to include an organic solvent step of treating the used superabsorbent polymers with an organic solvent or an aqueous solution thereof after the oxidizing agent treatment step S31 and before the drying step S33.

It should be noted that, as another embodiment, the citric acid removal step S32 may include, as the removal treatment step S40, after treating the used superabsorbent polymers with the above-described predetermined acid for removal, the above-described predetermined organic solvent, or an aqueous solution thereof, a reactivation step of further treating the treated used superabsorbent polymers with an aqueous solution containing an alkali metal ion source capable of supplying an alkali metal ion, that is, reactivating (neutralizing) the treated used superabsorbent polymers. In other words, the removal treatment step S40 may include a reactivation step after the acid step or the organic solvent step.

In this case, the superabsorbent polymers are inactivated with the acidic aqueous solution (inactivation step S30), and the inactivated superabsorbent polymers are neutralized with the aqueous solution containing the alkali metal ion source (removal treatment step S40), which makes it possible to obtain reactivated (neutralized) superabsorbent polymers. Therefore, it is not necessary to use a polyvalent metal ion for the inactivation, which makes it possible to suppress a situation where ash remains and absorption inhibition occurs.

Here, examples of the alkali metal ion in the alkali metal ion source include a lithium ion (Li ion), a sodium ion (Na ion), a potassium ion (K ion), and any combination thereof. The alkali metal ion source is not particularly limited as long as the alkali metal ion source is capable of supplying the above-described alkali metal ion, and examples thereof include a hydroxide of an alkali metal, a salt of a hydroxide of an alkali metal and an acid having a larger acid dissociation constant than an acid group in the superabsorbent polymers (hereinafter, also simply referred to as "salt"), and the like. As the acid dissociation constant, a value described in Handbook on Electrochemistry edited by the Electrochemical Society of Japan can be employed.

Examples of the hydroxide of an alkali metal include lithium hydroxide, sodium hydroxide, potassium hydroxide, and any combinations thereof. Incidentally, examples of the salt include an acidic salt and a basic salt. Examples of the hydroxide of an alkali metal in the salt include lithium hydroxide, sodium hydroxide, potassium hydroxide, and any combinations thereof. Examples of the acid in the salt include the acids exemplified in the inactivation step S30 (for example, hydrochloric acid and sulfuric acid), carbonic acid, and the like. Examples of the salt include lithium carbonate, sodium carbonate, potassium carbonate, lithium hydrogen carbonate, sodium hydrogen carbonate, potassium hydrogen carbonate, lithium chloride, sodium chloride, potassium chloride, and the like.

In the case of using the hydroxide of an alkali metal as the alkali metal ion source, the concentration of hydroxide ions of an alkaline aqueous solution is, for example, 0.1 to 5.0 mol/L, preferably 0.3 to 3.0 mol/L and more preferably 0.4 to 1.0 mol/L.

Furthermore, as another embodiment, in a case where the removal treatment step S40 is an organic solvent step, the citric acid removal step S32 may include a step of treating the used superabsorbent polymers with an aqueous solution of an organic solvent containing an alkali metal ion source capable of supplying an alkali metal ion. In other words, the organic solvent step of the removal treatment step S40 may include a reactivation step of reactivating the used superabsorbent polymers with an alkali metal ion source in addition to a dehydration and washing step of dehydrating and washing the used superabsorbent polymers with an organic solvent or an aqueous solution thereof. The dehydration and washing step and the reactivation step are performed at the same time as described above, which makes it possible to neutralize (reactivate) the used superabsorbent polymers with the alkali metal ion, while removing the citric acid from the used superabsorbent polymers and dehydrating the used superabsorbent polymers.

Therefore, in a case where reactivated (neutralized) superabsorbent polymers are required as the recycled superabsorbent polymers, the dehydration and washing step and the reactivation step are performed at the same time, which makes it possible to dehydrate the superabsorbent polymers without causing the superabsorbent polymers to absorb water and swell. This makes it possible to decrease the volume of the superabsorbent polymers to be reactivated and thus makes it possible to efficiently perform reactivation. In addition, a reactivation treatment can be performed on the superabsorbent polymers while the strength of a gel remains strong, which makes it possible to suppress the breakage of the gel due to stirring or the like during the reactivation treatment. In addition, there is no need to increase the size of a facility for reactivation or dehydration and washing, and the amount of the organic solvent used can be reduced. Furthermore, since the amount of moisture in the vicinity of the surfaces of the superabsorbent polymers can be made almost constant by the dehydration, the surfaces of the superabsorbent polymers can be dried relatively uniformly. This makes it possible to make the absorption performance of the recycled superabsorbent polymers relatively uniform and, consequently, to suppress a relative deterioration of the absorption performance.

Furthermore, as another embodiment, the citric acid removal step S32 may include a dehydration and washing step in which, in a case where the removal treatment step S40 is an acid step, after the removal treatment step S40, the used superabsorbent polymers are dehydrated and washed with an organic solvent or an aqueous solution thereof. Furthermore, as another embodiment, a dehydration and washing step of dehydrating and washing the used superabsorbent polymers with an organic solvent or an aqueous solution thereof may be included after an acid step is performed as the removal treatment step S40, and furthermore, a reactivation step of reactivating (neutralizing) the used superabsorbent polymers is performed. Including the dehydration and washing step makes it possible to more easily perform the subsequent drying step S33.

The temperature of the citric acid removal step S32 (removal treatment step S40) is a predetermined temperature, for example, room temperature (for example, 20°C) to 60°C and preferably 30°C to 50°C. When the temperature becomes low, there is a tendency that the time taken for removal becomes long, and, when the temperature becomes high, there are cases where the water-absorbing property of the superabsorbent polymers deteriorates due to dehydration condensation caused by the acid group of the superabsorbent polymers or the like. The time of the citric acid removal step S32 (removal treatment step S40) is a predetermined time, for example, 5 to 300 minutes.

The specific configuration of the citric acid removal apparatus 32 (removal treatment apparatus 40) that executes the citric acid removal step S32 (removal treatment step S40) is not particularly limited. Examples of the citric acid removal apparatus 32 (removal treatment step S40) include a mixer (standard mixer NS-P-S manufactured by Tatechs). The mixer includes a rotatable mixing tank. The superabsorbent polymers 301, the predetermined acid for removal, and the organic solvent or the aqueous solution thereof are supplied to the mixing tank and mixed by rotating the mixing tank. In the stage of the treatment of the citric acid removal step S32 (removal treatment step S40), since the superabsorbent polymers 301 are in a state of a gel having absorbed moisture, while not much, it is difficult to stir the gel with a stirring blade in a manner that the gel does not break and to evenly progress the reaction. This mixer is preferable because the superabsorbent polymers 301 can be evenly mixed with a predetermined aqueous solution without any stirring blades by rotating the mixing tank while rarely shearing the superabsorbent polymers 301. In the citric acid removal step S32 (removal treatment step S40), the superabsorbent polymers 301 and a predetermined aqueous solution are mixed with this mixer.

After that, the superabsorbent polymers 301 from which at least the citric acid has been removed are separated from the predetermined aqueous solution (solid-liquid separation) with a sieve (or mesh) provided in the citric acid removal step S32 (removal treatment step S40) and then supplied to the drying step S33 (drying apparatus 33) as superabsorbent polymers 302.

At the end stage of the treatment of the citric acid removal step S32 (removal treatment step S40), the superabsorbent polymers 302 are significantly dehydrated to be in a state where, for example, the water absorption ratio is about twice and the superabsorbent polymers 302 becomes as smooth as sand.

The drying step S33 is executed with the drying apparatus 33. In the drying step S33, the used superabsorbent polymers treated with the oxidizing agent are dried to generate recycled superabsorbent polymers. In the present embodiment, the superabsorbent polymers 302 are dried in a warming atmosphere while being stirred.

In the drying step S33, the drying temperature is, for example, room temperature (for example, 25°C) to 150°C and preferably 70°C to 120°C. When the drying temperature becomes low, there is a tendency that the drying time becomes long, and, when the drying temperature becomes high, there are cases where the water-absorbing property of the superabsorbent polymers deteriorates due to dehydrating condensation caused by the acid group of the superabsorbent polymers or the like. In the drying step S33, the drying time is, for example, 30 to 300 minutes. The drying step S33 may be performed under reduced pressure, for example, at 0.1 to 100 kPa from the viewpoint of accelerating drying.

The specific configuration of the drying apparatus 33 that executes the drying step S33 is not particularly limited as long as the superabsorbent polymers 302 can be dried. Examples of the drying apparatus 33 include a dryer with paddles (paddle dryer NPD-1.6W-G manufactured by Nara Machinery Co., Ltd.). The dryer with paddles includes a casing and rotatable biaxial shafts that are arranged in a room in the casing and extend parallel to each other in the axial direction. Each shaft includes a wedge-shaped heat transfer blade (paddle blade) that broadens in the radial direction, and the paddle blades of both shafts overlap each other when seen in the axial direction. The superabsorbent polymers 302 are supplied to the room, receives a hot air, and is dried while being stirred with the paddle blades in a high-temperature atmosphere. In the drying step S33, the superabsorbent polymers 302 are dried in a warming atmosphere with the dryer using paddles while being stirred so as to prevent the superabsorbent polymers 302 from being attached to each other.

It is preferable that the drying step S33 is performed so that the weight loss on drying of the superabsorbent polymers is preferably 15% or less (2.0 g, 105°C, and 3 hours). This is because the use of the superabsorbent polymers is taken into account. The above-described weight loss on drying is measured according to "7. Loss on Drying Test" of <2. General Tests, Processes and Apparatus> of "The Japanese Specifications of Sanitary Napkin Materials" attached as an accompanying sheet to "Regarding The Japanese Specifications of Sanitary Napkin Materials" notified by Ministry of Health, Labour and Welfare as PFSB/ELD Notification No.0325-(24) dated March 25, 2015.

After that, the dried superabsorbent polymers 302 are supplied to the foreign matter separation step S34 (foreign matter separation apparatus 34) as superabsorbent polymers 303.

The foreign matter separation step S34 is executed with the foreign matter separation apparatus 34. In the foreign matter separation step S34, foreign matters ares separated from the dried superabsorbent polymers 303. Examples of a method of separating the foreign matters include a method in which the foreign matters are separated based on a specific gravity difference in a gas or size selection by sieving. Examples of the foreign matters include pulp fibers.

The specific configuration of the foreign matter separation apparatus 34 that executes the foreign matter separation step S34 is not particularly limited as long as the foreign matters can be separated from the superabsorbent polymers 303. The foreign matter separation apparatus 34 is, for example, a cyclone separator and separates the superabsorbent polymers 303 and the foreign matters by centrifugation in a gas. This generates recycled superabsorbent polymers having a further reduced amount of the foreign matters.

Superabsorbent polymers derived from a used absorbent article are recycled in the above-described manner, thereby generating recycled superabsorbent polymers.

In the generated recycled superabsorbent polymers (here, reactivated (neutralized) superabsorbent polymers), as described in the following examples, the ash content is 35 mass% or less, preferably 32 mass% or less, and more preferably 31 mass% or less. In addition, in the generated recycled superabsorbent polymers, the viable bacteria count detected by the pour culture method is the detection limit or less. Here, it is considered that the amount of ash contained inside ordinary superabsorbent polymers that is not used after production (for example, a sodium polyacrylate crosslinked product) is about 30 mass% and thus the amount of ash contained inside the recycled superabsorbent polymers is also about 30 mass%. In this case, 30 mass% of the ash, that is, the ash inside the recycled superabsorbent polymers is considered to be derived from Na (sodium) of the sodium polyacrylate crosslinked product (it should be noted that ash on the surfaces of the superabsorbent polymers is considered to be an inorganic substance derived from excrement, silica originally attached to the surfaces or the like). Therefore, in the recycled superabsorbent polymers, the amount of unnecessary ash on the surfaces excluding the inside is small and is merely about 5 mass% (30 mass% from 35 mass% leaves 5 mass%) or less. The amount of the unnecessary ash is preferably 2 mass% or less, more preferably 1 mass% or less, and still more preferably 0.5 mass% or less. Furthermore, the recycling superabsorbent polymers has an extremely small viable bacteria count (the detection limit or less). Therefore, the purity of the recycled superabsorbent polymers is extremely high. Thus, these recycled superabsorbent polymers can be used as recycled superabsorbent polymers that are raw materials for producing superabsorbent polymers. In other words, these recycled superabsorbent polymers are recycled superabsorbent polymers that are recycled from used superabsorbent polymers derived from a used sanitary article and are raw materials for producing superabsorbent polymers.

In the method of producing recycled superabsorbent polymers according to the present embodiment, used superabsorbent polymers derived from a used sanitary article (for example, an absorbent article) are inactivated with the acidic solution (inactivation step S30), treated with the oxidizing agent under the acidic condition (oxidizing agent treatment step S31), and dried (drying step S33), thereby generating recycled superabsorbent polymers. That is, in the present method, a used superabsorbent polymer is dehydrated with an acidic solution to significantly decrease the volume of the used superabsorbent polymer, and then the used superabsorbent polymer is deodorized, decolorized, and sterilized with an oxidizing agent under an acidic condition while maintaining the dehydrated state, that is, the state where the volume is decreased. Such a decrease in the volume of the used superabsorbent polymer makes it possible to easily remove impurities (for example, an odorous substance, a colored substance, and bacteria) that are contained in the recycled superabsorbent polymer derived from a used sanitary article with an oxidizing agent and makes it possible to obtain a highly pure recycled superabsorbent polymer. Such a highly pure recycled superabsorbent polymer can be used as a part of a raw material in a predetermined method for forming a superabsorbent polymer to form highly pure high-quality superabsorbent polymers. Examples of the predetermined method of producing a superabsorbent polymers include methods as in Patent Literature 2 to 4 and a method in which superabsorbent polymers are produced using recycled superabsorbent polymers described below, that is, a method in which unused superabsorbent polymers are used as a part of raw materials of the superabsorbent polymers. This makes it possible to effectively use the used superabsorbent polymer derived from a used sanitary article for products for which a superabsorbent polymer is used.

In the present embodiment, as a preferable aspect, the foreign matters (for example, pulp fibers) are separated from the dried recycled superabsorbent polymers after the drying step S33 (foreign matter separation step S34). Therefore, compared with separation in a non-dry state such as separation in a solution, the foreign matter can be relatively easily separated from the recycled superabsorbent polymer, which makes it possible to obtain a more highly pure recycled superabsorbent polymer.

In the present embodiment, as a preferable aspect, the used superabsorbent polymers are inactivated with a solution containing citric acid (inactivation step S30). This does not only simply dehydrate the used superabsorbent polymer to significantly decrease the volume of the used superabsorbent polymer, but also makes it possible to easily remove a metal (for example, a metal derived from the human body that is contained in excrement) by a chelating effect of citric acid. Furthermore, in the present method, the citric acid in the used superabsorbent polymers are removed after the inactivation step S30 (citric acid removal step S32). This makes it possible to significantly suppress a chelating agent such as citric acid remaining in the recycled superabsorbent polymer. These make it possible to obtain a more highly pure recycled superabsorbent polymer. In particular, the chelating agent rarely remains in the recycled superabsorbent polymers, which makes it possible to significantly suppress the chelating agent significantly impairing a crosslinking reaction in a case where aqueous solution polymerization, crosslinking, or the like is performed in a method in which an unused superabsorbent polymers are reused as a part of raw materials of superabsorbent polymers.

In the present embodiment, as a preferable aspect, as the removal of the citric acid (citric acid removal step S32), the used superabsorbent polymers are treated with the acid for removal that does not form a chelating structure with a metal ion, the organic solvent that is miscible with water, or an aqueous solution thereof (removal treatment step S40). This makes it possible to wash off the citric acid on a surface of the used superabsorbent polymer while dehydrating the used superabsorbent polymer with the acid or the organic solvent. This makes it possible to more significantly suppress the chelating agent such as citric acid remaining in the recycled superabsorbent polymer.

In another embodiment, as the removal treatment step S40, the used superabsorbent polymers are treated with the acid for removal or an aqueous solution thereof during or after the oxidizing agent treatment step S31 (acid step). This makes it possible to further dehydrate the used superabsorbent polymer while removing the citric acid from the used superabsorbent polymer. This makes it possible to more easily execute the subsequent drying step S33 and thereby makes it possible to efficiently obtain highly pure recycled superabsorbent polymers.

In another embodiment, the acid for removal that is used in the removal treatment step S40 (acid step) is an acid having no carboxyl group or an acid having one carboxyl group in one molecule. This makes it possible in the removal treatment step to further dehydrate the used superabsorbent polymers while removing the citric acid from the used superabsorbent polymers.

In the present embodiment, as a preferable aspect, as the removal treatment step S40, the used superabsorbent polymers are treated with an aqueous solution of an organic solvent after the oxidizing agent treatment step S31 and before the drying step S33 (organic solvent step). This makes it possible to further dehydrate the used superabsorbent polymer while removing the citric acid from the used superabsorbent polymer. This makes it possible to more easily execute the subsequent drying step S33 and thereby makes it possible to efficiently obtain highly pure recycled superabsorbent polymers.

In another embodiment, in the removal treatment step S40 (organic solvent step), the used superabsorbent polymers are treated with an aqueous solution of an organic solvent containing an alkali metal ion source. This makes it possible to neutralize the used superabsorbent polymer with the alkali metal ion while removing the citric acid from the used superabsorbent polymer and dehydrating the used superabsorbent polymer. This makes it possible to use the recycled superabsorbent polymers as a neutralized highly pure raw material at the time of, for example, producing a superabsorbent polymers.

In another embodiment, the alkali metal ion source is a hydroxide of an alkali metal or a salt of a hydroxide of an alkali metal and an acid having a larger acid dissociation constant than an acid group of the superabsorbent polymers. This makes it possible to more reliably neutralize the used superabsorbent polymer with the alkali metal ion.

In the present embodiment, as a preferable aspect, in the oxidizing agent treatment step S31, the used superabsorbent polymers are treated with water containing ozone or a hydrogen peroxide solution as the oxidizing agent. This makes it possible to more easily remove impurities (for example, an odorous substance, a colored substance, and bacteria) contained in the recycled superabsorbent polymer derived from a used sanitary article, that is, makes it possible to more easily perform deodorization, decoloration, and sterilization. This makes it possible to obtain more highly pure recycled superabsorbent polymer.

In addition, in the recycled superabsorbent polymers derived from a used sanitary article (for example, an absorbent article) according to the present embodiment, the ash content is 35 mass% or less, and the viable bacteria count detected by a pour culture method is a detection limit or less. That is, the purity of the recycled superabsorbent polymers become high. This makes it possible to form highly pure high-quality superabsorbent polymers by incorporating such recycled superabsorbent polymers into at least one of raw materials and a semi-product in aqueous solution polymerization or crosslinking for producing the superabsorbent polymers.

Next, a method of producing superabsorbent polymers using recycled superabsorbent polymers that are recycled from used superabsorbent polymers derived from a used sanitary article and are raw materials for producing superabsorbent polymers will be described.

Among superabsorbent polymers, polyacrylate-based superabsorbent polymers are formed by, for example, an aqueous solution polymerization method. Examples of the aqueous solution polymerization method include the following methods (1) and (2) (Non-Patent Literature 1).
(1) Acrylic acid (raw material) is polymerized and crosslinked in an aqueous solution (polymerization initiator and crosslinking agent) to form a polyacrylic acid crosslinked product (semi-product), and then the polyacrylic acid crosslinked product is neutralized, thereby forming a sodium polyacrylate crosslinked product, that is, superabsorbent polymers. It is preferable that, after that, the formed superabsorbent polymers are surface-crosslinked with a surface crosslinking agent. In this case, the superabsorbent polymers before surface crosslinking can be referred to as a semi-product.
(2) Acrylic acid (raw material) is neutralized to form sodium acrylate (semi-product), and then sodium acrylate is polymerized and crosslinked in an aqueous solution (polymerization initiator and crosslinking agent), thereby forming a sodium polyacrylate crosslinked product, that is, superabsorbent polymers. It is preferable that, after that, the formed superabsorbent polymers are surface-crosslinked with a surface crosslinking agent. In this case as well, the superabsorbent polymers before surface crosslinking can be referred to as a semi-product.

Here, in the method (1), the above-described recycled superabsorbent polymers that have not been reactivated (neutralized) are incorporated into at least one of the acrylic acid (raw material) and the polyacrylic acid crosslinked product (semi-product), which makes it possible to produce superabsorbent polymers using recycled superabsorbent polymers. In addition, the above-described reactivated (neutralized) recycled superabsorbent polymers are incorporated into the superabsorbent polymers (semi-product) before the surface crosslinking, which makes it possible to produce superabsorbent polymers using recycled superabsorbent polymers.

In addition, in the method (2), the above-described recycled superabsorbent polymers that have not been reactivated (neutralized) are incorporated into the acrylic acid (raw material), which makes it possible to produce superabsorbent polymers using recycled superabsorbent polymers. In addition, the above-described reactivated (neutralized) recycled superabsorbent polymers are incorporated into the sodium acrylate (semi-product) or the superabsorbent polymers (semi-product) before the surface crosslinking, which makes it possible to produce superabsorbent polymers using recycled superabsorbent polymers.

Alternatively, the above-described recycled superabsorbent polymers that have not been reactivated (neutralized) and/or the above-described recycled superabsorbent polymers that have been reactivated (neutralized) are incorporated as the fine powder, fine particles, or water-absorbing resin (raw materials) of Patent Literature 2 to 4, which makes it possible to produce superabsorbent polymers using recycled superabsorbent polymers.

In the method of producing superabsorbent polymers using these recycled superabsorbent polymers, recycled superabsorbent polymers derived from a used sanitary article (for example, an absorbent article) are incorporated into at least one of raw materials and a semi-product, and superabsorbent polymers are produced by aqueous solution polymerization or crosslinking. At this time, since the recycled superabsorbent polymers are produced by the above-described method of producing recycled superabsorbent polymers, the purity thereof becomes high. This makes it possible in the present method to form highly pure high-quality superabsorbent polymers even when the above-described recycled superabsorbent polymers are used.

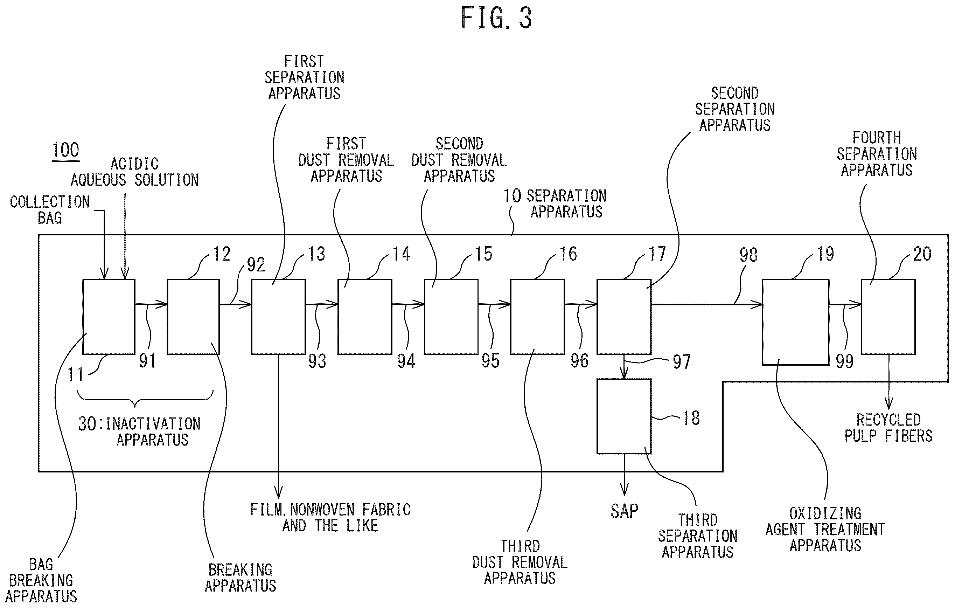

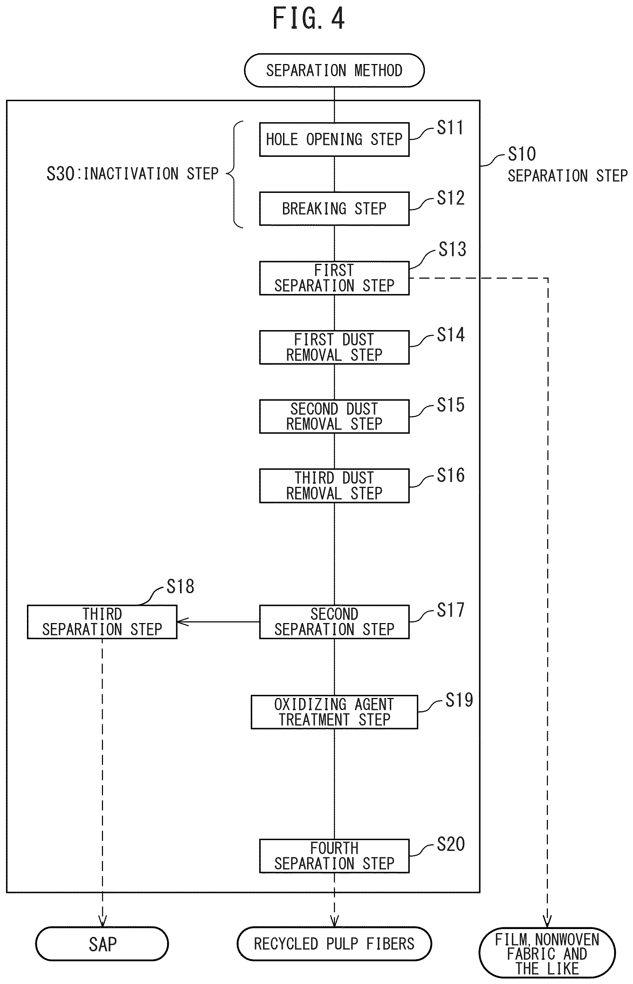

It should be noted that, in the method of producing recycled superabsorbent polymers derived from a used sanitary article (for example, absorbent article) according to the present embodiment, the method of extracting the superabsorbent polymers from the used sanitary article is not particularly limited, and any method can be employed. As such a method, for example, a method described below will be described with reference to FIG. 3 and FIG. 4.

FIG. 3 is a block diagram illustrating an example of a system 100 configured to separate members from a used absorbent article according to the present embodiment. The system 100 includes a separation apparatus 10 configured to separate a film, a nonwoven fabric, superabsorbent polymers (SAP), pulp fibers, and the like from a used absorbent article. Incidentally, FIG. 4 is a flow chart illustrating an example of a method of separating members from a used absorbent article according to the present embodiment. This method includes a separation step S10 of separating a film, a nonwoven fabric, superabsorbent polymers (SAP), and pulp fibers, and the like from a used absorbent article. In addition, as illustrated in FIG. 3, the separation apparatus 10 includes a bag breaking apparatus 11 to a fourth separation apparatus 20, and accordingly, the separation step S10 includes a hole opening step S11 to a fourth separation step S20 as illustrated in FIG.4. Hereinafter, each step will be specifically described.

It should be noted that, in the present embodiment, a used absorbent article is collected and acquired from the outside and is used for reuse (recycling). At that time, a plurality of used absorbent articles are enclosed in a collection bag so that excrement, fungi, or odors do not leak to the outside. Each used absorbent article in the collection bag is collected or the like in a state of being, mainly, rounded or folded such that the top sheet, onto which excrement is excreted, faces inward so as to prevent, for example, the excrement or the fungi from being exposed toward the surface and the odors from diffusing toward the surrounding.

The hole opening step S11 is executed with the bag breaking apparatus 11. The bag breaking apparatus 11 includes a solution tank that stores an inactivation aqueous solution containing an inactivating agent and a bag breaking blade that rotates in the solution tank. The bag breaking apparatus 11 opens holes in the collection bag put into the solution tank with the bag breaking blade in the inactivation aqueous solution. Thereby, a liquid mixture 91 of the collection bag into which the inactivating aqueous solution has intruded through the holes and the inactivating aqueous solution is generated. The inactivation aqueous solution inactivates the superabsorbent polymers in the used absorbent article in the collection bag. Hereinafter, a case where an acidic aqueous solution is used as the inactivation aqueous solution will be described as an example. A breaking step S12 is executed with a breaking apparatus 12. The breaking apparatus 12 includes a biaxial breaker (for example, a biaxial rotary breaker or the like). The breaking apparatus 12 breaks the collection bag including the used absorbent articles in the liquid mixture 91. Thereby, a liquid mixture 92 which contains broken matters of the collection bag including the used absorbent articles and the acidic aqueous solution is generated, and almost all of the superabsorbent polymers in the used absorbent articles are inactivated. Here, the broken matters contain the pulp fibers, the superabsorbent polymers, and other members (the films, the nonwoven fabrics, the collection bag, and the like).

A first separation step S13 is executed with a first separation apparatus 13. The first separation apparatus 13 includes a pulper separator having a stirring and separating tank that functions as a washing tank and a sieving tank. The first separation apparatus 13 separates the pulp fibers, the superabsorbent polymers, excrement, and the acidic aqueous solution from the liquid mixture 92 while stirring the liquid mixture 92 and removing excrement and the like from the broken matters. Thereby, a liquid mixture 93 containing the pulp fibers, the superabsorbent polymers, the excrement, and the acidic aqueous solution is generated, and the films and the nonwoven fabrics from the used absorbent articles, the materials of the collection bag, and the like are collected.

It should be noted that, since the used absorbent articles are is treated in the inactivation aqueous solution and the superabsorbent polymers is inactivated, the hole opening step S11 and the breaking step S12 (the bag breaking apparatus 11 and the breaking apparatus 12) can be referred to as the inactivation step S30 (inactivation apparatus 30). In addition, the breaking apparatus 12 may break the used absorbent articles in the collection bag in a gas (for example, in an air) instead of breaking the used absorbent articles in the inactivation aqueous solution. In such a case, the bag breaking apparatus 11 is not necessary. After the breaking, the broken matters and the inactivation aqueous solution in the breaking apparatus 12 are supplied to the first separation apparatus 13 (first separation step S13), and the superabsorbent polymers are inactivated. In such a case, the first separation apparatus 13 (first separation step S13) can be referred to as the inactivation step S30 (inactivation apparatus 30).

A first dust removal step S14 is executed with a first dust removal apparatus 14. The first dust removal apparatus 14 includes a screen separator and separates the liquid mixture 93 into the pulp fibers, the superabsorbent polymers, and the excrement in the acidic aqueous solution and other members (foreign matters) with a screen. Thereby, a liquid mixture 94 containing the pulp fibers, the superabsorbent polymers, the excrement, and the acidic aqueous solution, which decreases in weight by the amount of the foreign matters, is generated, and the other members are removed. A second dust removal step S15 is executed with a second dust removal apparatus 15. 8k The second dust removal apparatus 15 includes a screen separator and separates the liquid mixture 94 into the pulp fibers, the superabsorbent polymers, and the excrement in the acidic aqueous solution and other members (small foreign matters) with a finer screen than the first dust removal apparatus 14. Thereby, a liquid mixture 95 containing the pulp fibers, the superabsorbent polymers, the excrement, and the acidic aqueous solution, which further decreases in weight by the amount of the foreign matters, is generated, and the other members are further removed.

A third dust removal step S16 is executed with a third dust removal apparatus 16. The third dust removal apparatus 16 includes a cyclone separator and separates the liquid mixture 95 into the pulp fibers, the superabsorbent polymers, and the excrement in the acidic aqueous solution and other members (foreign matters having a heavy specific gravity) by centrifugation. Thereby, a liquid mixture 96 containing the pulp fibers, the superabsorbent polymers, the excrement, and the acidic aqueous solution, which further decreases in weight by the amount of the foreign matters, is generated, and the other members having a large specific gravity are removed.

It should be noted that, depending on the state of the liquid mixture 92 or the like (for example, the amounts or sizes of the foreign matters), at least one of the first dust removal apparatus 14 through the third dust removal apparatus 16 may not be provided.

A second separation step S17 is executed with a second separation apparatus 17. The second separation apparatus 17 includes a drum screen separator and separates the liquid mixture 96 into the superabsorbent polymers and the pulp fibers in the acidic aqueous solution with a drum screen. Thereby, a liquid mixture 97 containing the superabsorbent polymers, the excrement, and the acidic aqueous solution is generated, and the pulp fibers are removed as a mixture 98.

A third separation step S18 is executed with a third separation apparatus 18. The third separation apparatus 18 includes an inclined screen and separates the liquid mixture 97 into a solid containing the superabsorbent polymers and a liquid containing the excrement and the acidic aqueous solution with the screen. Thereby, superabsorbent polymers (SAP) are generated, and an acidic aqueous solution containing excrement or the like is removed.

An oxidizing agent treatment step S19 is executed with an oxidizing agent treatment apparatus 19. The oxidizing agent treatment apparatus 19 includes a treatment tank that stores an oxidizing agent aqueous solution and an oxidizing agent supply apparatus that supplies the oxidizing agent into the treatment tank. The oxidizing agent treatment apparatus 19 puts the mixture 98 into the treatment tank from the upper part or lower part of the treatment tank and mixes the mixture 98 with the oxidizing agent aqueous solution in the treatment tank. In addition, the oxidizing agent treatment apparatus 19 decomposes the superabsorbent polymers that are contained in the pulp fibers in the oxidizing agent aqueous solution with the oxidizing agent that is supplied from the lower part of the treatment tank with the oxidizing agent supply apparatus to solubilize the superabsorbent polymers in the oxidizing agent aqueous solution. Thereby, a liquid mixture 99 having the pulp fibers from which the superabsorbent polymers has been removed and the oxidizing agent aqueous solution containing a decomposition product of the superabsorbent polymers is generated. The oxidizing agent is an oxidizing agent capable of decomposing the superabsorbent polymers, examples thereof include ozone, and ozone is preferable due to high sterilizing power or bleaching power.

The ozone concentration in the oxidizing agent aqueous solution is preferably 1 to 50 ppm by mass. When the concentration is too low, the superabsorbent polymers cannot be completely solubilized, and there is a risk that the superabsorbent polymers may remain in the pulp fibers. When the concentration is too high, there is a risk that the pulp fibers may be damaged. The treatment time with ozone is short when the ozone concentration in the oxidizing agent aqueous solution is high and is long when the ozone concentration is low. The treatment time is typically 5 to 120 minutes. The product of the ozone concentration (ppm) in the oxidizing agent aqueous solution and the treatment time (minutes) (hereinafter, also referred to as "CT value") is preferably 100 to 6000 ppm·minute. When the CT value is too small, the superabsorbent polymers cannot be completely solubilized, and, when the CT value is too large, there is a risk that the pulp fibers may be damaged.

The fourth separation step S20 is executed with the fourth separation apparatus 20. The fourth separation apparatus 20 includes a screen separator and separates the liquid mixture 99 into the pulp fibers and the oxidizing agent aqueous solution with a screen. Thereby, pulp fibers are generated, and the oxidizing agent aqueous solution containing the decomposition product of the superabsorbent polymers is removed.

### <Ash>

It should be noted that a method of measuring ash is as described below. The ash refers to the amount of an inorganic or non-combustible residue that remains after the organic substance is ashed. The ash is measured according to "5. Total Ash Test" of "2. General tests, Processes and Apparatus" of The Japanese Specifications of Sanitary Napkin Materials. That is, the ash is measured as described below. A platinum, quartz, or porcelain crucible is ignited at 500°C to 550°C in advance for one hour and left to be cooled, and then the mass thereof is precisely weighed. Two to four grams of a sample is collected and put in the crucible, the mass thereof is precisely weighed, the lid of the crucible is removed or shifted as necessary, the sample is weakly heated in the beginning, the temperature is gradually increased to ignite the sample at 500°C to 550°C for four hours or longer to ash the sample until no carbide remains. After the sample is left to be cooled, the mass is precisely weighed. The residue is ashed again until the amount becomes constant and left to be cooled, and then the mass thereof is precisely weighed and regarded as the amount of ash (mass%).

### <Detection of viable bacteria>

Viable bacteria can be detected by a pour culture method. Examples of the viable bacteria include Bacillus cereus, Bacillus subtilis, Staphylococcus aureus, Pseudomonas aeruginosa, non-fermenting bacilli of glucose, and Aeromonas. When the above-described bacteria are not detected by the pour culture method, the recycled superabsorbent polymers is less likely to cause bacteremia, endocarditis, respiratory infections, food poisoning, eye infections, and the like, and users can use the recycled superabsorbent polymers with peace of mind.

The pour culture method is performed as described below.
(1) 500 g of an aqueous dispersion liquid of recycled superabsorbent polymers having a solid content concentration of 5.0 mass% is prepared in a 1-liter beaker.

In a case where the recycled superabsorbent polymers are present in a dry state, the aqueous dispersion liquid can be formed by mixing the recycled superabsorbent polymers (25.0 g as a solid content) and deionized water (amount that makes the total amount 500.0 g). In addition, in a case where the recycled superabsorbent polymers are present as an aqueous solution (for example, in a case where the recycled superabsorbent polymers are collected as an aqueous solution in the method of producing recycled superabsorbent polymers) and the solid content concentration of the recycled superabsorbent polymers is 5.0 mass% or more, the aqueous dispersion liquid of recycled superabsorbent polymers having a solid content concentration of 5.0 mass% can be prepared by adding deionized water to the aqueous solution or the like.

Furthermore, in a case where the recycled superabsorbent polymers are present as an aqueous solution and the solid content concentration of the recycled superabsorbent polymers is less than 5.0 mass%, the solid content concentration of the recycled superabsorbent polymers can be adjusted to 5.0 mass% by filtration or the inoculum dose of a serially diluted sample, which will be described below, can be increased using the above-described aqueous solution itself as the aqueous dispersion liquid (for example, in a case where the solid content concentration of the recycled superabsorbent polymers is 2.5 mass%, the inoculum dose is doubled).
(2) The aqueous dispersion liquid is stirred for 15 minutes at a rotation speed of 300 rpm using an overhead stirrer.
(3) 50 mL of the aqueous dispersion liquid stirred with the overhead stirrer is put into a sterile bag with a filter (manufactured by LMS, a sterile bag with a filter for a homogenizer) and stirred for five minutes.
(4) The filtered aqueous dispersion liquid that has been filtered with the sterile bag with a filter is dispensed into a sterilized test tube, serially diluted 10 times up to 10⁻⁹, and dispensed into a sterilized test tube, thereby preparing a serially diluted sample.
(5) The viable bacteria count is measured by the pour culture method.

Specifically, 1 mL of a serially diluted sample and a standard agar medium (manufactured by Nihon Pharmaceutical Co., Ltd., 396-00175 SCD agar medium "DAIGO" for general bacteria inspection, 15 to 20 g) are put into a petri dish and pour-cultured at 35°C for 48 hours.
(6) As the viable bacteria count, the number of colonies that have grown after culturing is counted. It should be noted that, in a case where the numbers of the colonies are zero in all of the serially diluted samples serially diluted 10 times up to 10⁻⁹, a target bacterium is determined to be "not detected", that is, the viable bacteria count detected by the pour culture method is the detection limit or less. In other words, the viable bacteria count is 0 cfu/g.
(7) In a case where the colonies of enteric bacteria or viable bacteria are formed after culturing, the type of bacteria can be identified. The identification can be performed by a biochemical property inspection method.

### Examples

Hereinafter, the present invention will be described based on examples, but the present invention is not limited to the examples.

### (1) Regarding oxidizing agent treatment

The effect of an oxidizing agent treatment was evaluated.

### (a) Sample

Superabsorbent polymers separated from a used diaper that had been actually used (corresponding to the separation step S10) was used. The superabsorbent polymers were almost a sodium polyacrylate crosslinked product. The separated superabsorbent polymers are inactivated with an acidic aqueous solution (citric acid concentration of 1%) (corresponding to the inactivation step S30 = corresponding to the hole opening step S11 + the breaking step S12).

### (b) Evaluation method

(i) In the sample of the example, 1 g of the (inactivated) superabsorbent polymers separated from the used diaper were immersed in ozone water (ozone concentration of 1 ppm) having a mass 10 times the mass of the superabsorbent polymers under an acidic condition (citric acid concentration of mass 1%) (corresponding to the oxidizing agent treatment step S31) for two minutes. On the other hand, in a sample of a comparative example, 1 g of an (inactivated) superabsorbent polymers separated from a used diaper were not immersed in ozone water.
(ii) After that, the superabsorbent polymers as the sample of the example and the superabsorbent polymers as the sample of the comparative example were both treated in an aqueous solution containing methanol and sodium hydroxide (corresponding to the citric acid removal step S32). This dehydrated and reactivated (neutralized) each of the superabsorbent polymers while removing the citric acid from each of the superabsorbent polymers.
(iii) After that, the superabsorbent polymers as the sample of the example and the superabsorbent polymers as the sample of the comparative example were both dried at 105°C for 60 minutes (corresponding to the drying step S33).
(iv) For the obtained superabsorbent polymers as the sample of the example and the obtained superabsorbent polymers as the sample of the comparative example, ashes and the number of viable bacteria were measured, respectively. The evaluation results are shown in Table 1.

### (c) Evaluation result

The amount of ash in the superabsorbent polymers as the sample of the example was 30.3 mass%, was reduced by about 10 mass% compared with 40.9 mass% that was the amount of ash in the superabsorbent polymers as the sample of the comparative example, and became almost the same as the amount of ash (about 30 mass%) that is contained in a general superabsorbent polymers (sodium polyacrylate crosslinked product) that is not used after production. Therefore, it was found that there was almost no substance that became ash (1 mass% or less) on the surfaces of the superabsorbent polymers as the sample of the example. That is, since an inorganic substance derived from excrement or silica or the like originally attached to the surfaces can be considered as a substance that becomes ash on the surfaces of the superabsorbent polymers, it was found that there was almost no inorganic substance derived from excrement or silica on the surfaces of the superabsorbent polymers as the sample of the example. In other words, in the sample of the example, it can be said that the amount of ash on the surfaces of the superabsorbent polymers is 1 mass% or less. Furthermore, the number of viable bacteria in the superabsorbent polymers as the sample of the example was the detection limit or less (0 (zero) cfu/g) and exhibited an extremely low value compared with 3400 cfu/g that was the number of viable bacteria in the superabsorbent polymers as the sample of the comparative example. Therefore, it was found that the purity of the superabsorbent polymers as the sample of the example, that is, the recycled superabsorbent polymers was extremely high.

**[Table 1]**

| | Ash (mass%) | Viable bacteria count (cfu/g) |
|---|---|---|
| Examples | 30.3 | 0 |
| Comparative Example | 40.9 | 3400 |

The present invention is not limited to each of the above-described embodiments and can be appropriately combined, modified, or the like without departing from the aspect and purport of the present invention.

### REFERENCE SIGNS LIST

S30: Inactivation step
S31: Oxidizing agent treatment step
S33: Drying step

This application is a divisional application of European patent application number 20861009.7 (the "parent" application) also published under number 4001354. The original claims of the parent application are repeated as statements below in the present specification and form part of the content of this divisional application as filed.

Clause 1 A method of producing a recycled superabsorbent polymer that is a raw material for producing a superabsorbent polymer from a used superabsorbent polymer derived from a used sanitary article, the method comprising:
an inactivation step of inactivating the used superabsorbent polymer with an acidic solution;
an oxidizing agent treatment step of treating the used superabsorbent polymer inactivated with the acidic solution with an oxidizing agent under an acidic condition; and
a drying step of drying the used superabsorbent polymer treated with the oxidizing agent to generate the recycled superabsorbent polymer.

Clause 2 The method according to clause 1, further comprising:
a foreign matter separation step of separating a foreign matter from the recycled superabsorbent polymer after the drying step.

Clause 3 The method according to clauses 1 or 2, wherein
the acidic solution is a solution containing citric acid,
the method further comprising, after the inactivation step and before the drying step:
   a citric acid removal step of removing the citric acid in the used superabsorbent polymer.

Clause 4 The method according to clause 3, wherein
the citric acid removal step includes a removal treatment step of treating the used superabsorbent polymer with an acid for removal that is an acid that does not form a chelating structure with a metal ion, an organic solvent that is miscible with water, or an aqueous solution thereof.

Clause 5 The method according to clause 4, wherein
the removal treatment step includes, during or after the oxidizing agent treatment step, an acid step of treating the used superabsorbent polymer with the acid for removal or an aqueous solution thereof.

Clause 6 The method according to clauses 4 or 5, wherein
the acid for removal is an acid having no carboxyl group or an acid having one carboxyl group in one molecule.

Clause 7 The method according to any one of clauses 4 to 6, wherein
the removal treatment step includes, after the oxidizing agent treatment step and before the drying step, an organic solvent step of treating the used superabsorbent polymer with an aqueous solution of the organic solvent.

Clause 8 The method according to clause 7, wherein
the organic solvent step includes a step of treating the used superabsorbent polymer with an aqueous solution of the organic solvent containing an alkali metal ion source capable of supplying an alkali metal ion.

Clause 9 The method according to clause 8, wherein
the alkali metal ion source is a hydroxide of an alkali metal, or a salt of a hydroxide of an alkali metal and an acid having a larger acid dissociation constant than an acid group of the superabsorbent polymer.

Clause 10 The method according to any one of clauses 1 to 9, wherein,
in the oxidizing agent treatment step, the oxidizing agent contains water containing ozone or a hydrogen peroxide solution.

Clause 11 A method of producing a superabsorbent polymer using a recycled superabsorbent polymer that is recycled from a used superabsorbent polymer derived from a used sanitary article and is a raw material for producing a superabsorbent polymer, the method comprising:
a step of producing the recycled superabsorbent polymer by the method according to any one of claims 1 to 10; and
a step of executing aqueous solution polymerization or crosslinking for producing the superabsorbent polymer by incorporating the recycled superabsorbent polymer into at least one of a raw material and a semi-product for the production in the aqueous solution polymerization or crosslinking.

Clause 12 A recycled superabsorbent polymer that is recycled from a used superabsorbent polymer derived from a used sanitary article and is a raw material for producing a superabsorbent polymer, in which an ash content is 35 mass% or less and a viable bacteria count detected by a pour culture method is a detection limit or less.

## Claims

1. A recycled superabsorbent polymer that is recycled from a used superabsorbent polymer derived from a used sanitary article and is a raw material for producing a superabsorbent polymer, in which an ash content is 35 mass% or less as measured by the method described in paragraph [0107] of the description, and a viable bacteria count detected by a pour culture method is a detection limit or less as measured by the method described in paragraph [0109] of the description.

2. The recycled superabsorbent polymer according to claim 1, including a liquid substance inside.

3. The recycled superabsorbent polymer according to claim 2, wherein the liquid substance includes excrement.

4. The recycled superabsorbent polymer according to any one of claims 1 to 3, including bacteria and other organic substances derived from excrement inside.
